# EUROPÄISCHE PATENTSCHRIFT

(11) **EP 2 250 147 B1**
(45) Veröffentlichungstag und Bekanntmachung des Hinweises auf die Patenterteilung: **12.10.2011**
(21) Anmeldenummer: 09701315.5
(22) Anmeldetag: 07.01.2009
(51) Int. Cl.: C07C 231/12

(54) **VERFAHREN ZUR HERSTELLUNG VON ACYLGLYCINATEN MITTELS DIREKTOXIDATION**
METHOD FOR THE PRODUCTION OF ACYL GYLCINATES BY MEANS OF DIRECT OXIDATION
PROCÉDÉ DE PRODUCTION D'ACYLGLYCINATES PAR OXYDATION DIRECTE

(30) Priorität: 10.01.2008 DE 102008003825
(43) Veröffentlichungstag der Anmeldung: 17.11.2010
(73) Patentinhaber: Clariant Finance (BVI) Limited, Road Town, Tortola (VG)
(72) Erfinder: KLUG, Peter, 63762 Grossostheim (DE); STANKOWIAK, Achim, 84503 Altötting (DE); FRANKE, Oliver, 81671 München (DE); SCHERL, Franz-Xaver, 84508 Burgkirchen (DE); PRÜSSE, Ulf, 38118 Braunschweig (DE); DECKER, Nadine, 15537 Erkner (DE); VORLOP, Klaus-Dieter, 38102 Braunschweig (DE)
(74) Vertreter: Paczkowski, Marcus
(86) Internationale Anmeldenummer: PCT/EP2009/000034
(87) Internationale Veröffentlichungsnummer: WO 2009/087086

(56) Entgegenhaltungen:
- WO-A-2008/000648
- WO-A-2008/000671
- WO-A-2008/019807
- JP-A- 11 246 473

## Beschreibung

Aminosäuretenside sind in der Waschmittel- und Kosmetikindustrie weit verbreitet. Sie zählen zu der Gruppe der milden Cotenside und werden meist zur Verbesserung des Schaumvolumens und der Milde der Formulierungen eingesetzt. Sie wurden in der Vergangenheit hauptsächlich über die Umsetzung von Aminosäuren mit aktivierten Fettsäurederivaten, speziell Fettsäurechloriden, synthetisiert wie z. B. in US 6,703,517 oder US 2005/0085651 A1 für Acylglycinate der Formel (IIa) beschrieben (s. Schema 1). 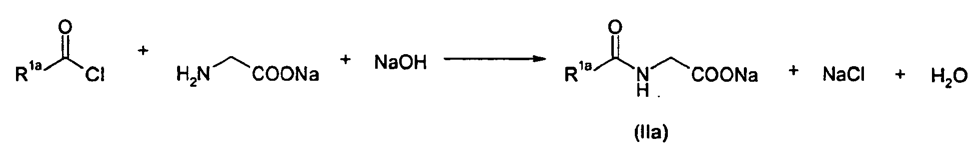 R^{1a} ist ein gesättigter oder ungesättigter Fettsäurerest mit 8 bis 22 Kohlenstoffatomen

### Schema 1 Herstellung von Natrium-Acylglycinaten nach dem Stand der Technik.

Dieses Verfahren nach dem Stand der Technik benötigt mit dem Fettsäurechlorid einen relativ teuren und reaktiven Rohstoff und weist außerdem den Nachteil auf, dass pro ein mol Aminosäuretensid, d. h. Verbindung der Formel (IIa), ein mol Kochsalz NaCl gebildet wird. Dieses gelangt in das Reaktionsabwasser und stellt dort für biologische Kläranlagen ein Problem dar, da Kochsalz die Reinigungsleistung solcher Anlagen beeinträchtigen kann.

JP 11 246473 A offenbart in Paragraph [0039-0040] ein Verfahren zur Herstellung von Dodecansäure-glycinamid, dadurch gekennzeichnet, dass Dodecansäure-2-ethanolamid in wässriger Lösung mit 4 Äquivalenten Acetaledehyd in Gegenwart von Essigsäure, 0.2mol% Co(OAc)₂-tetrahydrat sowie 0.2mol% RuCl₃-n-hydrat umgesetzt wird.

Es bestand also ein Bedarf an Verfahren zur Herstellung von Aminosäuretensiden und hier speziell Aminosäuretensiden auf Basis der Aminosäure Glycin, so genannte Acylglycinate und deren protonierte Basissäuren, die die obigen Nachteile nicht aufweisen.

Überraschend wurde gefunden, dass acylierte Glycine und deren Salze, so genannte Acylglycinatsalze oder kurz Acylgylcinate, alternativ zu der üblichen Fettsäurechloridroute nach dem Stand der Technik auch durch direkte Oxidation von Fettsäuremonoethanolamiden mit Luftsauerstoff oder Reinsauerstoff mittels Übergangsmetallkatalysatoren zugänglich sind.

Gegenstand der vorliegenden Erfindung ist daher ein Verfahren zur Herstellung von Acylglycinatsalzen der Formel (II) 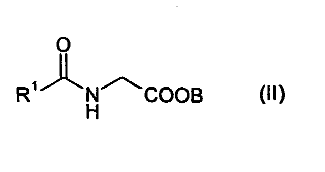 worin
R¹ einen gesättigten linearen oder verzweigten Alkylrest mit 1 bis 21 Kohlenstoffatomen oder einen ein- oder mehrfach ungesättigten linearen oder verzweigten Alkenylrest mit 2 bis 21 Kohlenstoffatomen und
B ein Kation ist,
bedeutet, und/oder der entsprechenden protonierten Acylglycinsäuren, dadurch gekennzeichnet, dass ein oder mehrere Fettsäuremonoethanolamide der Formel
(I) 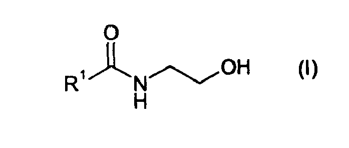 worin R¹ die oben angegebene Bedeutung besitzt,
mit Sauerstoff in Gegenwart eines gegebenenfalls geträgerten Bimetallkatalysators bestehend aus Gold und einem Metall der Gruppe VIII des

Periodensystems im alkalischen Medium zu einem oder mehreren Acylglycinatsalzen der Formel (II) oxidiert werden und im Fall der Herstellung der protonierten Acylglycinsäuren das oder die Acylglycinatsalze der Formel (II) zusätzlich mit einer Säure umgesetzt werden.

Im Rahmen der vorliegenden Erfindung werden unter den Metallen der Gruppe VIII des Periodensystems die Metalle Fe, Ru, Os, Co, Rh, Ir, Ni, Pd und Pt verstanden.

Bei dem erfindungsgemäßen Verfahren wird gegenüber der Verwendung des Fettsäurechlorids mit einem deutlich preisgünstigeren Rohstoff, dem Fettsäuremonoethanolamid der Formel (I), gestartet. Außerdem wird bei der Herstellung der Acylglycinate der Formel (II) kein Salz gebildet (s. Schema 2). 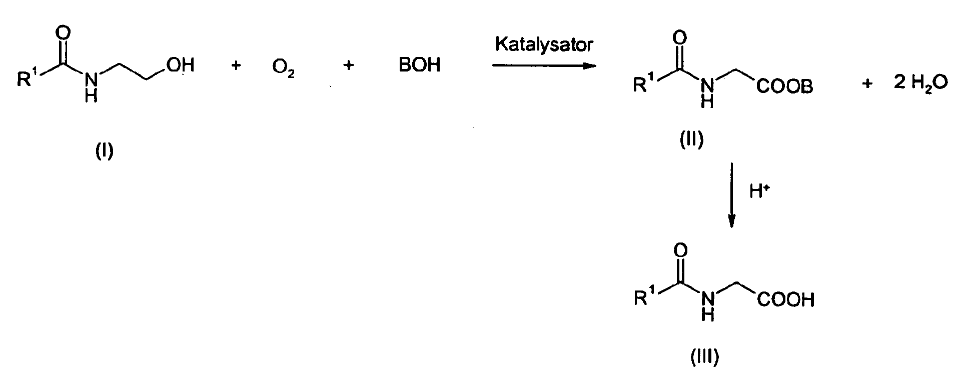 R¹ und B haben die oben angegebenen Bedeutungen

### Schema 2 Herstellung von Acylglycinaten und/oder der entsprechenden protonierten Acylglycinsäuren nach dem erfindungsgemäßen Verfahren.

Als Fettsäuremonoethanolamide können Monoethanolamide von gesättigten, unverzweigten oder verzweigten Fettsäuren mit 2-22 Kohlenstoffatomen (d. h. R¹ = C₁ bis C₂₁) oder von ein- oder mehrfach ungesättigten, unverzweigten oder verzweigten Fettsäuren mit 3-22 Kohlenstoffatomen (d.h. R¹ = C₂ bis C₂₁) eingesetzt werden.

Bevorzugt sind Fettsäuremonoethanolamide mit 8-18 Kohlenstoffatomen im Fettsäurerest bzw. Acylrest R¹CO-, d. h. R¹ ist in diesem Fall ein gesättigter linearer oder verzweigter Alkylrest mit 7 bis 17 Kohlenstoffatomen oder ein einoder mehrfach ungesättigter linearer oder verzweigter Alkenylrest mit 7 bis 17 Kohlenstoffatomen. Besonders bevorzugt sind Laurinsäuremonoethanolamid, Myristinsäuremonoethanolamid, Caprylsäuremonoethanolamid, Caprinsäuremonoethanolamid, Palmitinsäuremonoethanolamid, Stearinsäuremonoethanolamid oder Isostearinsäuremonoethanolamid. Hierbei können auch Amide basierend auf Kettenschnitten bzw. Mischungen dieser Fettsäuremonoethanolamide eingesetzt werden, bevorzugt Cocosfettsäuremonoethanolamid.

Unter den gesättigten und ungesättigten Fettsäuremonoethanolamiden sind die gesättigten Fettsäuremonoethanolamide bevorzugt.

Bei dem Kation B handelt es sich vorzugsweise um Alkalimetallkationen ausgewählt aus Kationen der Alkalimetalle Li, Na, K, Rb und Cs. Besonders bevorzugt sind die Kationen der Alkalimetalle Na und K.

Die Bimetallkatalysatoren sind gemischte Katalysatoren, die neben Gold noch ein Metall aus der Gruppe VIII enthalten. Die Bimetallkatalysatoren sind demnach vorzugsweise Goldkatalysatoren, die zusätzlich mit einem der Metalle aus der Gruppe VIII dotiert sind. Besonders bevorzugt ist die Dotierung mit Platin oder Palladium.

Vorzugsweise sind die Metalle der Bimetallkatalysatoren bzw. sind die Bimetallkatalysatoren auf einem Träger aufgebracht. Bevorzugte Träger sind Aktivkohle und oxidische Träger, vorzugsweise oxidische Träger. Als oxidische Träger wiederum bevorzugt sind Titandioxid, Cerdioxid oder Aluminiumoxid, besonders bevorzugt bestehen die oxidischen Träger aus Titandioxid. Solche Katalysatoren können nach den bekannten Methoden wie Incipient Wetness (IW) oder Deposition-Precipitation (DP) wie z. B. in L. Prati, G. Martra, Gold Bull. 39 (1999) 96 und S. Biella, G.L. Castiglioni, C. Fumagalli, L. Prati, M. Rossi, Catalysis Today 72 (2002) 43-49 oder L. Prati, F. Porta, Applied catalysis A: General 291 (2005) 199-203 beschrieben, hergestellt werden.

In einer weiteren bevorzugten Ausführungsform der Erfindung enthalten die geträgerten Bimetallkatalysatoren 0,1 bis 5 Gew.-% und vorzugsweise 0,5 bis 3 Gew.-% Gold.

Weiterhin bevorzugt enthalten die geträgerten Bimetallkatalysatoren 0,1 bis 3 Gew.-% und vorzugsweise 0,1 bis 2 Gew.-% eines Metalls der Gruppe VIII, vorzugsweise Platin oder Palladium.

Besonders bevorzugt enthalten die geträgerten Bimetallkatalysatoren 0,1 bis 5 Gew.-%, insbesondere 0,5 bis 3 Gew.%, Gold und 0,1 bis 3 Gew.-%, insbesondere 0,1 bis 2 Gew.-%, eines Gruppe VIII-Metalls, bevorzugt Platin oder Palladium, bezogen auf die Summe von Trägermaterial und Bimetallkatalysator. Das bevorzugte Gewichtsverhältnis Gold/Gruppe VIII Metall, insbesondere Gold/Platin oder Gold/Palladium, beträgt 70 : 30 bis 95 : 5. Gleiche Mengenverhältnisse gelten auch bei Verwendung von Gold und anderer Metalle der Gruppe VIII als Platin oder Palladium.

Die Teilchengröße des Bimetallkatalysators ist vorzugsweise von 1 bis 50 nm und besonders bevorzugt von 2 bis 10 nm. Diese Bimetallkatalysatoren werden im Rahmen der vorliegenden Erfindung auch als Nanogoldkatalysatoren bezeichnet.

Als Basen können Carbonate, Hydroxide oder Oxide in dem erfindungsgemäßen Verfahren verwendet werden. Bevorzugt sind die Hydroxide BOH.

Das erfindungsgemäße Verfahren wird vorzugsweise in Wasser durchgeführt.

Die Oxidationsreaktion wird vorzugsweise bei einer Temperatur von 30 bis 200 °C, besonders bevorzugt zwischen 80 und 150 °C, durchgeführt.

Der pH-Wert bei der Oxidation liegt bevorzugt zwischen 8 und 13, besonders bevorzugt zwischen 9 und 12.

Der Druck bei der Oxidationsreaktion ist vorzugsweise im Vergleich zu Atmosphärendruck erhöht.

Bei der Reaktion im alkalischen Medium entstehen zunächst die Alkalisalze (B = Li, Na, K, Rb, Cs) der acylierten Glycine mit 2 bis 22 Kohlenstoffatomen im Acylrest, bevorzugt mit 8 bis 18 Kohlenstoffatomen, bevorzugt die Natrium- oder Kaliumsalze. Besonders bevorzugt ist das Verfahren für Natriumcocoylglycinat und Kaliumcocoylglycinat. Durch Ansäuern mit anorganischen Säuren kann aus den Lösungen dann das acylierte Glycin erhalten werden. Bevorzugte Säuren sind Salz- und Schwefelsäure.

In einer weiteren Ausführungsform der Erfindung wurde der Tatsache Rechnung getragen, dass längerkettige (≥ C₈) Fettsäuremonoethanolamide, d. h. Fettsäuremonoethanolamide mit 8 oder mehr Kohlenstoffatomen im Acylrest R¹CO-, speziell Laurinsäuremonoethanolamid und Cocosfettsäuremonoethanolamid, für eine ausreichende Oxidationsreaktion ohne Zusatz geeigneter Lösemittel nicht ausreichend im Reaktionsmedium Wasser löslich sind. Somit würde der Vorteil der Kochsalz-freien Produktion der Zielsubstanzen in diesem Fall durch den zusätzlichen Einsatz von Lösemitteln wieder teilweise aufgehoben.

Es wurde nun gefunden, dass Fettsäuremonoethanolamide mit 8 oder mehr Kohlenstoffatomen im Acylrest R¹CO-, speziell Laurinsäuremonoethanolamid und Cocosfettsäuremonoethanolamid, in Lösungen von Alkalisalzen der Acylglycinate bzw. Fettsäureglycinate, insbesondere der Alkalisalze von Laurinsäure- oder Cocosfettsäureglycinaten, löslich sind. Dadurch ergibt sich eine elegante, lösemittelfreie Reaktionsführung dadurch, dass man eine Lösung von Fettsäuremonoethanolamid im Zielprodukt Acylglycinatsalz, vorzugsweise Natriumacylglycinat, herstellt (dies kann durch Rückmischung der fertigen Reaktionslösung mit Fettsäuremonoethanolamid erfolgen) und diese Mischung der katalytischen Oxidation unterwirft. Dabei wird das enthaltene Monoethanolamid oxidiert und wiederum eine Lösung eines Alkalisalzes (B = Li, Na, K, Rb, Cs) der Acylgylcinsäure der Formel (III) in Wasser erzeugt. Besonders bevorzugt sind hierbei die Natrium- und Kaliumsalze (B = Na, K).

Anschließend kann wiederum aus der alkalischen Reaktionslösung mittels geeigneter Säuren die Acylglycinsäure der Formel (III) freigesetzt werden. Bevorzugte Säuren sind Salzsäure und Schwefelsäure.

In einer bevorzugten Ausführungsform der Erfindung wird somit eine Lösung enthaltend ein oder mehrere Fettsäuremonoethanolamide der Formel (I) und ein oder mehrere Acylglycinate der Formel (II) der Oxidation unterworfen.

In dieser bevorzugten Ausführungsform der Erfindung werden die Fettsäuremonoethanolamide mittels Sauerstoff und einem gegebenenfalls geträgerten Bimetallkatalysator bestehend aus Gold und einem Metall der Gruppe VIII im alkalischen Medium zu Lösungen von Acylglycinaten oxidiert, wobei vor Beginn der Oxidationsreaktion eine Lösung des Fettsäuremonoethanolamids in einem Alkalisalz eines Acylglycinats vorliegt und diese Mischung in Wasser der Oxidationsreaktion unterworfen wird.

In dieser bevorzugten Ausführungsform der Erfindung beträgt das Massenverhältnis zwischen Fettsäuremonoethanolamid der Formel (I) und Acylglycinat der Formel (II) zu Beginn der Reaktion zwischen und 1 : 10 und 3 : 1, bevorzugt zwischen 1 : 2 und 2 : 1. Der Gesamtmassenanteil an Fettsäuremonoethanolamid der Formel (I) und Acylglycinat der Formel (II) beträgt zwischen 15 und 50 %, bevorzugt zwischen 20 und 40 %, besonders bevorzugt zwischen 25 und 35 %.

Das erfindungsgemäße Verfahren ergibt vorzugsweise Lösungen von Acylglycinaten der Formel (II) mit nur noch geringen Restgehalten an Fettsäuremonoethanolamid von < 10 Gew.-%, bevorzugt < 5 Gew.-%, besonders bevorzugt < 2 Gew.-%.

Die folgenden Beispiele illustrieren die Erfindung:

### Beispiel 1: Verfahren zur Herstellung von Glycinaten unter Verwendung von Goldkatalysatoren

In einen 2-Liter-Druckautoklaven mit Begasungsrührer werden 1 Liter einer wässrigen Lösung, enthaltend 15 Gew.-% Cocosfettsäuremonoethanolamid und 15 Gew.-% Natriumcocoylglycinat, gegeben. Diese Mischung ist bis 80 °C klar und flüssig. Die Menge an Natriumcocoylglycinat kann aus einem vorausgegangenen Ansatz entnommen werden bzw. aus einem vorausgegangenen Oxidationsansatz im Reaktor verbleiben. Nach Zugabe von 5 g eines Nanogoldkatalysators (0,9 Gew.-% Gold und 0,1 Gew.-% Platin auf Titandioxid, Teilchengröße 4-8 nm) wird die Suspension mit Natronlauge auf pH 12 eingestellt und auf 80 °C aufgeheizt. Nach Erreichen der Reaktionstemperatur wird die Reaktionslösung mit Sauerstoff auf einen Druck von 9 bar aufgepresst und durch Nachpressen auf diesem Druck gehalten. Während der gesamten Reaktionszeit wird mittels eines Autotitrators der pH-Wert der Mischung mit Natronlauge auf 12 gehalten. Nach 6 Stunden wird der Reaktor abgekühlt, entspannt und der Katalysator durch Filtration von der Reaktionslösung abgetrennt. Die Lösung zeigt einen Restgehalt von < 2 Gew.-% Cocosmonoethanolamid und ca. 32 Gew.-% Natriumcocoylglycinat.

### Beispiel 2: Verfahren zur Herstellung von Glycinaten unter Verwendung von Goldkatalysatoren

In einen 2-Liter-Druckautoklaven mit Begasungsrührer werden 1 Liter einer wässrigen Lösung, enthaltend 15 Gew.-% Cocosfettsäuremonoethanolamid und 15 Gew.-% Kaliumcocoylglycinat, gegeben. Diese Mischung ist bis 80 °C klar und flüssig. Die Menge an Kaliumcocoylglycinat kann aus einem vorausgegangenen Ansatz entnommen werden bzw. aus einem vorausgegangenen Oxidationsansatz im Reaktor verbleiben. Nach Zugabe von 5 g eines Nanogoldkatalysators (0,9 Gew.-% Gold und 0,1 Gew.-% Platin auf Titandioxid, Teilchengröße 4-8 nm) wird die Suspension mit Natronlauge auf pH 12 eingestellt und auf 80 °C aufgeheizt. Nach Erreichen der Reaktionstemperatur wird die Reaktionslösung mit Sauerstoff auf einen Druck von 9 bar aufgepresst und durch Nachpressen auf diesem Druck gehalten. Während der gesamten Reaktionszeit wird mittels eines Autotitrators der pH-Wert der Mischung mit Kalilauge auf 12 gehalten. Nach 6 Stunden wird der Reaktor abgekühlt, entspannt und der Katalysator durch Filtration von der Reaktionslösung abgetrennt. Die Lösung zeigt einen Restgehalt von < 1 Gew.-% Cocosmonoethanolamid und ca. 33 Gew.-% Kaliumcocoylglycinat.

## Patentansprüche

1. Verfahren zur Herstellung von Acylglycinatsalzen der Formel (II) 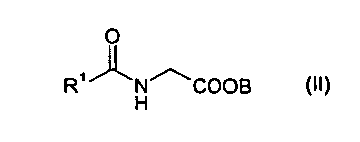 worin
R¹ einen gesättigten linearen oder verzweigten Alkylrest mit 1 bis 21 Kohlenstoffatomen oder einen ein- oder mehrfach ungesättigten linearen oder verzweigten Alkenylrest mit 2 bis 21 Kohlenstoffatomen und
B ein Kation ist,
bedeutet, und/oder der entsprechenden protonierten Acylglycinsäuren, **dadurch gekennzeichnet, dass** ein oder mehrere Fettsäuremonoethanolamide der Formel (I) 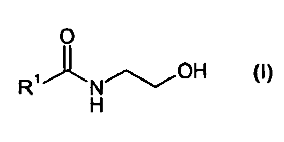 worin R¹ die oben angegebene Bedeutung besitzt,
mit Sauerstoff in Gegenwart eines gegebenenfalls geträgerten Bimetallkatalysators bestehend aus Gold und einem Metall der Gruppe VIII des Periodensystems im alkalischen Medium zu einem oder mehreren Acylglycinatsalzen der Formel (II) oxidiert werden und im Fall der Herstellung der protonierten Acylglycinsäuren das oder die Acylglycinatsalze der Formel (II) zusätzlich mit einer Säure umgesetzt werden.

2. Verfahren nach Anspruch 1, **dadurch gekennzeichnet, dass** ein oder mehrere Fettsäuremonoethanolamide der Formel (I), worin der oder die Reste R¹ gesättigte lineare oder verzweigte Alkylreste mit 7 bis 17 Kohlenstoffatomen oder ein- oder mehrfach ungesättigte lineare oder verzweigte Alkenylreste mit 7 bis 17 Kohlenstoffatomen sind, in der Reaktion verwendet werden.

3. Verfahren nach Anspruch 1 oder 2, **dadurch gekennzeichnet, dass** das oder die Fettsäuremonoethanolamide der Formel (I) ausgewählt sind aus Laurinsäuremonoethanolamid, Myristinsäuremonoethanolamid, Caprylsäuremonoethanolamid, Caprinsäuremonoethanolamid, Palmitinsäuremonoethanolamid, Stearinsäuremonoethanolamid, Isostearinsäuremonoethanolamid und Cocosfettsäuremonoethanolamid.

4. Verfahren nach einem oder mehreren der Ansprüche 1 bis 3, **dadurch gekennzeichnet, dass** der Bimetallkatalysator auf einem Träger aufgebracht ist.

5. Verfahren nach Anspruch 4, **dadurch gekennzeichnet, dass** der Bimetallkatalysator auf einem oxidischen Träger aufgebracht ist.

6. Verfahren nach Anspruch 5, **dadurch gekennzeichnet, dass** der oxidische Träger aus Titandioxid besteht.

7. Verfahren nach einem oder mehreren der Ansprüche 4 bis 6, **dadurch gekennzeichnet, dass** der geträgerte Bimetallkatalysator 0,1 bis 5 Gew.-% Gold, bevorzugt 0,5 bis 3 Gew.-% Gold, enthält.

8. Verfahren nach einem oder mehreren der Ansprüche 4 bis 7, **dadurch gekennzeichnet, dass** der geträgerte Bimetallkatalysator 0,1 bis 3 Gew.-% und vorzugsweise 0,1 bis 2 Gew.-% eines Metalls der Gruppe VIII, vorzugsweise Platin oder Palladium, enthält.

9. Verfahren nach einem oder mehreren der Ansprüche 1 bis 8, **dadurch gekennzeichnet, dass** die Teilchengröße des Bimetallkatalysators von 1 bis 50 nm und vorzugsweise von 2 bis 10 nm ist.

10. Verfahren nach einem oder mehreren der Ansprüche 1 bis 9, **dadurch gekennzeichnet, dass** eine Lösung enthaltend ein oder mehrere Fettsäuremonoethanolamide der Formel (I) und ein oder mehrere Acylglycinate der Formel (II) der Oxidation unterworfen werden.

## Claims

1. A process for preparing acylglycinate salts of the formula (II) 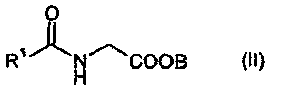 where
R¹ is a saturated linear or branched alkyl radical having from 1 to 21 carbon atoms or a monounsaturated or polyunsaturated linear or branched alkenyl radical having from 2 to 21 carbon atoms and
B is a cation,
and/or the corresponding protonated acylglycine acids, which comprises oxidizing one or more fatty acid monoethanolamides of the formula (I) 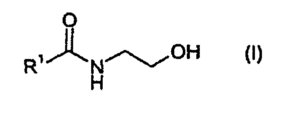 where R¹ is as defined above,
by means of oxygen in the presence of an optionally supported bimetal catalyst consisting of gold and a metal from group VIII of the Periodic Table of Elements in alkaline medium to form one or more acylglycinate salts of the formula (II) and, in the case of the preparation of the protonated acylglycine acids, additionally reacting the acylglycinate salt or salts of the formula (II) with an acid.

2. The process as claimed in claim 1, wherein one or more fatty acid monoethanolamides of the formula (I) in which the radical or radicals R¹ is/are saturated linear or branched alkyl radicals having from 7 to 17 carbon atoms or monounsaturated or polyunsaturated linear or branched alkenyl radicals having from 7 to 17 carbon atoms are used in the reaction.

3. The process as claimed in claim 1 or 2, wherein the fatty acid monoethanolamide or monoethanolamides of the formula (I) is/are selected from among lauric acid monoethanolamide, myristic acid monoethanolamide, caprylic acid monoethanolamide, capric acid monoethanolamide, palmitic acid monoethanolamide, stearic acid monoethanolamide, isostearic acid monoethanolamide and coconut fatty acid monoethanolamide.

4. The process as claimed in one or more of claims 1 to 3, wherein the bimetal catalyst has been applied to a support.

5. The process as claimed in claim 4, wherein the bimetal catalyst has been applied to an oxidic support.

6. The process as claimed in claim 5, wherein the oxidic support comprises titanium dioxide.

7. The process as claimed in one or more of claims 4 to 6, wherein the supported bimetal catalyst contains from 0.1 to 5% by weight of gold, preferably from 0.5 to 3% by weight of gold.

8. The process as claimed in one or more of claims 4 to 7, wherein the supported bimetal catalyst contains from 0.1 to 3% by weight and preferably from 0.1 to 2% by weight of a metal from group VIII, preferably platinum or palladium.

9. The process as claimed in one or more of claims 1 to 8, wherein the particle size of the bimetal catalyst is from 1 to 50 nm and preferably from 2 to 10 nm.

10. The process as claimed in one or more of claims 1 to 9, wherein a solution containing one or more fatty acid monoethanolamides of the formula (I) and one or more acylglycinates of the formula (II) is subjected to the oxidation.

## Revendications

1. Procédé pour la préparation de sels d'acylglycinate de formule (II) 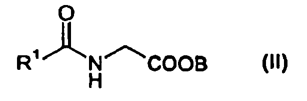 où
R¹ signifie un radical alkyle saturé, linéaire ou
ramifié, comprenant 1 à 21 atomes de carbone ou un radical alcényle monoinsaturé ou polyinsaturé, linéaire ou ramifié comprenant 2 à 21 atomes de carbone et
B signifie un cation
et/ou des acides acylglyciniques protonés correspondants, **caractérisé en ce qu'**on oxyde un ou plusieurs monoéthanolamides d'acide gras de formule (I) 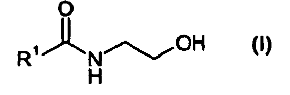 où R¹ a la signification indiquée ci-dessus,
avec de l'oxygène, en présence d'un catalyseur bimétallique le cas échéant supporté, constitué d'or et d'un métal du groupe VIII du système périodique, en milieu alcalin, en un ou plusieurs sels d'acylglycinate de formule (II) et, dans le cas de la préparation des acides acylglyciniques protonés, on transforme en outre le ou les sels d'acylglycinate de formule (II) avec un acide.

2. Procédé selon la revendication 1, **caractérisé en ce qu'**un ou plusieurs monoéthanolamides d'acide gras de formule (I), où le ou les radicaux R¹ représentent des radicaux alkyle saturés, linéaires ou ramifiés comprenant 7 à 17 atomes de carbone ou des radicaux alcényle monoinsaturés ou polyinsaturés, linéaires ou ramifiés, comprenant 7 à 17 atomes de carbone, sont utilisés dans la réaction.

3. Procédé selon la revendication 1 ou 2, **caractérisé en ce que** le ou les monoéthanolamides d'acide gras de formule (I) sont choisis parmi le monoéthanolamide de l'acide laurique, le monoéthanolamide de l'acide myristique, le monoéthanolamide de l'acide caprylique, le monoéthanolamide de l'acide caprique, le monoéthanolamide de l'acide palmitique, le monoéthanolamide de l'acide stéarique, le monoéthanolamide de l'acide isostéarique et le monoéthanolamide d'acide gras de coco.

4. Procédé selon l'une ou plusieurs des revendications 1 à 3, **caractérisé en ce que** le catalyseur bimétallique est appliqué sur un support.

5. Procédé selon la revendication 4, **caractérisé en ce que** le catalyseur bimétallique est appliqué sur un support oxyde.

6. Procédé selon la revendication 5, **caractérisé en ce que** le support oxyde est constitué de dioxyde de titane.

7. Procédé selon l'une ou plusieurs des revendications 4 à 6, **caractérisé en ce que** le catalyseur bimétallique supporté contient 0,1 à 5% en poids d'or, de préférence 0,5 à 3% en poids d'or.

8. Procédé selon l'une ou plusieurs des revendications 4 à 7, **caractérisé en ce que** le catalyseur bimétallique supporté contient 0,1 à 3% en poids et de préférence 0,1 à 2% en poids d'un métal du groupe VIII, de préférence le platine ou le palladium.

9. Procédé selon l'une ou plusieurs des revendications 1 à 8, **caractérisé en ce que** la grosseur des particules du catalyseur bimétallique est de 1 à 50 nm et de préférence de 2 à 10 nm.

10. Procédé selon l'une ou plusieurs des revendications 1 à 9, **caractérisé en ce qu'**une solution contenant un ou plusieurs monoéthanolamides d'acide gras de formule (I) et un ou plusieurs acylglycinates de formule (II) est soumise à l'oxydation.
